Europäisches Patentamt

European Patent Office

Office européen des brevets

(19)

(11) Publication number: **0 134 264**
**B1**

(12) **EUROPEAN PATENT SPECIFICATION**

(45) Date of publication of patent specification: **04.02.87**

(21) Application number: **83107806.8**

(22) Date of filing: **08.08.83**

(51) Int. Cl.⁴: **C 07 C 79/04,** C 07 C 76/02 //
B01J19/12

(54) Preparation of nitroalkanes with actinic light.

(43) Date of publication of application:
**20.03.85 Bulletin 85/12**

(45) Publication of the grant of the patent:
**04.02.87 Bulletin 87/06**

(84) Designated Contracting States:
**BE CH DE FR GB IT LI LU NL SE**

(56) References cited:
**AT-B- 244 318**
**DE-B-1 037 438**
**US-A-3 113 975**
**US-A-3 120 478**
**US-A-3 272 874**
**US-A-4 329 523**

(73) Proprietor: **THE DOW CHEMICAL COMPANY**
**2030 Dow Center Abbott Road P.O. Box 1967**
**Midland, MI 48640 (US)**

(72) Inventor: **Hayes, William Verne**
**202 Hackberry, No. 4403**
**Clute, TX 77531 (US)**
Inventor: **Jones, Mark Berry**
**108 Ironwood**
**Lake Jackson, TX 77566 (US)**

(74) Representative: **Weickmann, Heinrich, Dipl.-Ing.**
**et al**
**Patentanwälte Dipl.-Ing. H.Weickmann Dipl.-**
**Phys.Dr. K.Fincke Dipl.-Ing. F.A.Weickmann**
**Dipl.-Chem. B. Huber Dr.-Ing. H. Liska Dipl.-**
**Phys.Dr. J. Prechtel Postfach 860820**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Nitroalkanes are an essential stabilizing ingredients employed in 1,1,1-trichloroethane when it is used in vapor degreasing and cold cleaning. All manufacturers throughout the world add nitromethane and/or nitroethane to their commercial 1,1,1-trichloroethane-based solvents. Normally nitroalkanes are manufactured by a vapor phase nitration of the alkane with either nitric acid or $NO_2$. There is a mixture of products formed due to carbon-carbon scission. Thus, for example, when propane is nitrated, the products include 1-nitropropane, 2-nitropropane, nitroethane and nitromethane. Because of the oxidative conditions other oxygen-containing compounds are also produced, e.g. aldehydes, acids, and carbon oxides. Patents disclosing such a process are U.S. 2,844,634 and 2,905,724.

Improvements in these vapor phase nitrations are claimed by employing the nitric acid or nitrogen oxides together with oxygenated sulfur compounds, e.g. $SO_2$, $H_2SO_4$, (U.S. 3,272,874) and by conducting the nitration in the presence of ozone (U.S. 3,113,975).

Other processes involve nitration of paraffins by nitrogen peroxide $(NO_2)_2$ in the presence of oxygen (air) under pressure at 150°—330°C (U.S. 3,780,115); reacting an olefin with nitric acid in the presence of a lower aliphatic monocarboxylic acid anhydride to produce a nitroester and subsequently reducing with an alkali borohydride to form the nitroalkane (U.S. 3,706,808) and reacting organic amines with ozone (U.S. 3,377,387).

The present process is an improvement which reduces the amount of by-products and obtains improved yields of the desired products by conducting the nitration of paraffins by employing nitric acid at lower temperatures and pressures in the presence of ultraviolet light.

It has now been discovered that the lower alkanes containing from 1 to 4 carbon atoms can be nitrated to yield the analogous nitroalkanes and/or a nitroalkane having fewer carbon atoms than the starting alkane. The process reacts nitric acid with an alkane in the vapor phase in the presence of actinic radiation. Thus, for example, one obtains a mixture of nitromethane and nitroethane by conducting the reaction of nitric acid and ethane in the presence of ultraviolet light, having wavelengths within the range of 3200 Å to 14000 Å, at atmospheric pressure and a temperature of 130°C at a contact time of about 8 seconds.

The reaction to produce nitroalkanes at lower pressures and temperatures is conducted by exposing the lower alkane and nitric acid reactants in the vapor phase to actinic light. Temperatures are desirably in the range of 100° to 150°C and the pressure is desirably 0 to 700 kPa gauge. A molar ratio of lower alkane to nitric acid of from 0.75 to 3.0 moles of alkane per mole of acid and a contact time of 4 to 10 seconds are employed.

Preferred temperature, pressure, contact time and molar ratios of reactants are from about 125° to about 140°C, from 0 to 350 kPa gauge, 6.5 to 9 seconds and from 1.2 to 2.5 moles of alkanes per mole of acid.

Although not required, it is preferred to conduct the reaction in a glass reactor so the reactant may be easily exposed to actinic light. Alternatively, the reactor may have a light well for the admission of the light. Conventional ultraviolet lamps are a suitable source of light.

### Example 1

In a representative example of the invention nitric acid was fed by a positive displacement metering pump through a vaporizing preheater, mixed with nitrogen diluent and a gaseous alkane, in this case, ethane. The molar ratio of ethane to $HNO_3$ was 1.2/1. The mixed gas stream was then introduced into a reactor equipped with a light well (essentially a glass window which admits an ultraviolet light source) and steam-jacketed walls. The steam pressure was regulated for temperature control. At 125—130°C, atmospheric pressure, and 7.8 seconds contact time, 18.1 percent of the ethane being fed was converted to 15.3 percent nitromethane and 43.5 percent nitroethane. The effluent from the reactor was scrubbed with water in a packed trickle bed column and the water analyzed by gas chromatography for the nitro-alkanes. Ethane conversion was determined by taking gas samples of the mixed feed and reactor effluent and analyzing by gas chromatograph.

### Example 2

A source of U.V. light (Conrad-Hanovia lamp, #679A—0360) was employed using Vycor® Pyrex® and quartz glass reactors, which selectively transmitted certain sections of the spectrum, and employed as in Example 1 an ethane/$HNO_3$ mol ratio of 1.2/1 at 130°C, atmospheric pressure and 7.8 seconds reaction time. Table I shows the conditions and results of these runs using ethane and nitric acid.

### TABLE I

| Reactor Material | UV Wavelength range | % Conversion of $C_2H_6$ | % Selectivity to NM* | NE* |
|---|---|---|---|---|
| Quartz | 2200Å—14000Å | 8.8 | 12.6 | 35.9 |
| Vycor | 2800Å—14000Å | 11.8 | 12.6 | 37.9 |
| Pyrex | 3200Å—14000Å | 18.9 | 14.3 | 40.5 |

*NM = nitromethane, NE = nitroethane

### Example 3

In another experiment run under the same conditions, methane and 30 percent nitric acid were employed and the molar ratio of the reactants was varied to determine the effect on conversion and selectivity. The ratios used and results are shown in Table II.

### TABLE II

| $CH_4/HNO_3$ mol ratio | % Conversion of $CH_4$ | % Selectivity to $CH_3NO_2$ |
|---|---|---|
| 0.89/1 | 19.0 | 18.9 |
| 1.06/1 | 16.1 | 23.6 |
| 1.48/1 | 14.6 | 21.5 |
| 2.97/1 | 3.8 | 51.8 |

### Example 4

The contact time was varied to determine its effect on conversion and selectivity employing a temperature of 130°C and atmospheric pressure. The $HNO_3$, employed as 68 percent nitric acid, was used at a ratio of 1.2 moles methane per mole of acid. Table III shows the results.

### TABLE III

| Contact Time (seconds) | % Conversion of $CH_4$ | % Selectivity to NM |
|---|---|---|
| 5.0 | 4.3 | 50.7 |
| 6.5 | 13.1 | 21.2 |
| 7.0 | 15.9 | 17.6 |
| 9.4 | 17.3 | 17.0 |

### Example 5

The contact time was varied at a mol ratio of 1.76 moles ethane per mol of $HNO_3$ employing a temperature of 130°C and a pressure of 0 psig. Results are shown in Table IV.

### TABLE IV

| Contact Time (seconds) | % Conversion of $C_2H_6$ | % Selectivity to NM | NE |
|---|---|---|---|
| 8.4 | 5.8 | 18.0 | 51.6 |
| 9.2 | 7.4 | 16.5 | 45.5 |
| 10.2 | 10.0 | 15.4 | 41.9 |

### Example 6

Under the same conditions of mol ratio and pressure as in Example 5, ethane and $HNO_3$ were reacted at substantially the same contact time but at different temperatures. Results are shown in Table V.

3

TABLE V

| Temperature (°C) | Contact Time (seconds) | % Conversion of $C_2H_6$ | % Selectivity to NM | NE |
|---|---|---|---|---|
| 128 | 8.4 | 6.0 | 17.5 | 48.5 |
| 135 | 8.6 | 19.7 | 8.9 | 22.1 |

**Claims**

1. A process of making nitroalkanes which comprises reacting $HNO_3$ and a lower alkane in the vapor phase at a temperature below 150°C and a pressure of less than 700 kPa in the presence of actinic light.

2. The process of Claim 1 wherein the mol ratio of lower alkane to $HNO_3$ is 0.75 to 3 moles alkane per mol of $HNO_3$.

3. The process of Claim 2 wherein the reaction temperature is maintained at 125 to 140°C.

4. The process of Claim 2 wherein the pressure of the reaction is maintained at from 0 to 350 kPa.

5. The process of Claim 2 wherein the contact time is from 4 to 10 seconds.

6. The process of Claims 1, 2, 3, 4 or 5 wherein the light employed has a wavelength within the range of from 2200 to 14000Å.

7. The process of Claim 6 wherein the high intensity light employed has a wavelength within the range of from 3200 to 14000Å.

8. A process of making nitroalkanes by reacting $HNO_3$ and a $C_1$ to $C_4$ alkane in the vapor phase, the improvement of which comprises, conducting the reaction

(a) at a pressure of less than 700 kPa;

(b) at a temperature of less than 150°C; and

(c) in the presence of actinic light.


**Patentansprüche**

1. Verfahren zur Herstellung von Nitroalkanen, dadurch gekennzeichnet, daß man $HNO_3$ und ein niederes Alkan in der Dampfphase bei einer Temperatur unterhalb von 150°C und einem Druck von weniger als 700 kPa in Gegenwart von aktinischem Licht umsetzt.

2. Verfahren nach Anspruch 1, dadurch gekennzeichnet, daß das Molverhältnis von niederem Alkan zu $HNO_3$ 0,75 bis 3 Mole Alkan pro Mol $HNO_3$ beträgt.

3. Verfahren nach Anspruch 2, dadurch gekennzeichnet, daß die Reaktionstemperatur bei 125 bis 140°C gehalten wird.

4. Verfahren nach Anspruch 2, dadurch gekennzeichnet, daß der Druck der Reaktion bei 0 bis 350 kPa gehalten wird.

5. Verfahren nach Anspruch 2, dadurch gekennzeichnet, daß die Kontaktzeit 4 bis 10 Sekunden beträgt.

6. Verfahren nach Anspruch 1, 2, 3, 4 oder 5, dadurch gekennzeichnet, daß das verwendete Licht eine Wellenlänge im Bereich von 2200 bis 14000 Å besitzt.

7. Verfahren nach Anspruch 6, dadurch gekennzeichnet, daß das verwendete Licht hoher Intensität eine Wellenlänge im Bereich von 3200 bis 14000 Å besitzt.

8. Verfahren zur Herstellung von Nitroalkanen durch Umsetzung von $HNO_3$ und einem $C_1$ bis $C_4$-Alkan in der Dampfphase, dadurch gekennzeichnet, daß man die Reaktion durchführt bei

(a) einem Druck von weniger als 700 kPa;

(b) einer Temperatur von weniger als 150°C; und

(c) in Gegenwart von aktinischem Licht.


**Revendications**

1. Procédé de production de nitroalcanes, qui consiste à faire réagir $HNO_3$ et un alcane inférieur, en phase vapeur, à une température inférieure à 150°C et sous une pression inférieure à 700 KPa, en présence d'une lumière actinique.

2. Procédé selon la revendication 1, dans lequel le rapport molaire de l'alcane inférieur au $HNO_3$ est de 0,75 à 3 moles d'alcane per mole de $HNO_3$.

3. Procédé selon la revendication 2, dans lequel on maintient la température de réaction entre 125 et 140°C.

4. Procédé selon la revendication 2, dans lequel on maintient la pression de réaction entre 0 et 350 KPa.

5. Procédé selon la revendication 2, dans lequel la durée de contact est de 4 à 10 secondes.

6. Procédé selon la revendication 1, 2, 3, 4 ou 5, dans lequel la lumière utilisée présente une longueur d'onde située dans le domaine allant de 2200 à 14.000 Å.

**0 134 264**

7. Procédé selon la revendication 6, dans lequel la lumière à haute intensite utilisée présente une longueur d'onde située dans le domaine allant de 3200 à 14.000 Å.

8. Procédé de production de nitroalcanes par réaction en phase vapeur entre $HNO_3$ et un alcane en $C_1$—$C_4$, perfectionné en ce qu'il consiste à effectuer la réaction:

    (a)  à une pression inférieure à 700 kPa;

    (b)  à une température inférieure à 150°C; et

    (c)  en présence de lumière actinique.

5